# EUROPÄISCHE PATENTSCHRIFT

(11) **EP 1 309 305 B1**
(45) Veröffentlichungstag und Bekanntmachung des Hinweises auf die Patenterteilung: **27.04.2005**
(21) Anmeldenummer: 01962632.4
(22) Anmeldetag: 14.08.2001
(51) Int. Cl.: A61K 7/00, A61K 7/48

(54) **KOSMETISCHER BASISKOMPLEX**
COSMETIC BASE COMPLEX
COMPLEXE DE BASE COSMETIQUE

(30) Priorität: 15.08.2000 DE 10042576
(43) Veröffentlichungstag der Anmeldung: 14.05.2003
(73) Patentinhaber: Coty B.V., 2031 CC Haarlem (NL)
(72) Erfinder: GOLZ-BERNER, Karin, MC-98000 Monaco (MC); ZASTROW, Leonhard, MC-98000 Monaco (MC); OLIVIER, Doucet, F-06230 Villefranche s/Mer (FR)
(74) Vertreter: Walter, Wolf-Jürgen
(86) Internationale Anmeldenummer: PCT/DE2001/003071
(87) Internationale Veröffentlichungsnummer: WO 2002/013770

(56) Entgegenhaltungen:
- EP-A- 0 875 236
- EP-A- 1 106 174
- EP-A- 1 159 882
- DE-A- 19 941 819

## Beschreibung

Die Erfindung betrifft einen kosmetischen Basiskomplex mit Extrakten von Früchten und Milchprodukten.

Es sind bereits eine Reihe von Vorschlägen gemacht worden, Früchte oder Fruchtextrakte einschließlich maritimer Pflanzen und Algen in kosmetischen Produkten zu verarbeiten (DE-C-3515231 Hafnia-Extrakt; DE-A-19824727 Catechin und grüner Tee).

Es ist weiterhin bekannt, Milch oder aufbereitete Milchprodukte in kosmetische Produkte einzubringen. So ist aus der EP-A-839519 eine protein- und vitaminhaltige Kosmetikzusammensetzung bekannt, die 5-90 Gew-% Säugermilch, 1-6 Gew-% Protein und Hefeglucan enthält.

Der Erfindung liegt die Aufgabe zugrunde, einen Basiskomplex aus einer Kombination von Frucht- und Milchproduktextrakten mit spezieller Haut- und Farbwirksamkeit zu entwickeln.

Erfindungsgemäß bereitgestellt wird ein kosmetischer Basiskomplex, bestehend aus einer Gelgrundlage und darin enthalten einen verkapselten Extrakt einer wäßrigen Extraktion der Ananas-Frucht und den Rückstand einer wäßrigen Extraktion von Joghurt, wobei das Verhältnis Ananas-Extrakt zu Joghurtextraktionsrückstand im Bereich von 20:80 bis 80:20 liegt, bezogen auf das Gewicht,
und wobei die Extraktionen jeweils im Temperaturbereich von 10 bis 30 °C erfolgten.

Der Extrakt der Ananas-Frucht wird durch Extraktion mit Wasser bei einer Temperatur im Bereich von 10 bis 30 °C, vorzugsweise 15 bis 25 °C erhalten. Dabei geht man von zerkleinerten frischen Früchten aus, die unter Bewegung über einen Zeitraum von 5 bis 24 Stunden mit Wasser in Kontakt gebracht werden. Der Extrakt wird anschließend filtriert, und dann verkapselt. Die Verkapselung kann vorzugsweise mit Phospholipiden zu üblichen Liposomen erfolgen. Dabei wird das bei der Extraktion erhaltene Endopeptidasen-Gemisch der Bromelain-Gruppe, wie Bromelin, Ananase, Inflamen, Extranase usw. sowie weitere Enzyme in die Kapseln im wesentlichen vollständig aufgenommen. Zusätzlich enthalten in dem Ananas-Extrakt sind Vitamine, wie Vitamin C, B1, B2, B6, Mineralstoffe wie hohe Kaliumgehalte bei niedrigen Natriumgehalten, Chlor, Calcium, Phosphor, Magnesium Eisen, Mangan, Kupfer, Zink, Iod sowie Serotonin.

Wie bereits ausgeführt, können übliche Liposome als Transportsystem für den Ananas-Extrakt eingesetzt werden. Liposome sind vollständig geschlossene Lipid-Bilayer-Membranen, die ein wäßriges Volumen eingeschlossen enthalten. Liposome können unilamellare Vesikel sein (die eine Einzelmembran-Bilayer besitzen) oder multilamellare Vesikel (Onion-ähnliche Strukturen, gekennzeichnet durch Mehrfachmembran-Bilayer, von denen jede von der nächsten durch eine wäßrige Schicht getrennt ist). Die Bilayer besteht aus zwei Lipid-Monolayern, die einen hydrophoben "Schwanz"-Bereich und einen hydrophilen "Kopf"-Bereich haben. Die Struktur der Membran-Bilayer ist so, daß die hydrophoben (unpolaren) "Schwänze" der Lipidmonolayer sich in Richtung des Zentrums der Bilayer orientieren, während sich die hydrophilen "Köpfe" in Richtung der wäßrigen Phase orientieren.

Die Herstellung von Liposomen, aus gesättigten und ungesättigten Lipiden, ist in sehr vielen Patenten beschrieben worden, ebenso deren Einsatz als Transportsystem. Vorteilhaft werden für die vorliegende Erfindung Phospholipide als Liposombildner eingesetzt, wobei die Herstellung auf übliche Weise erfolgt unter gleichzeitiger Aufnahme des Extraktes in die Liposomen.

Als Ausgangsstoff für die Ananas-Extraktion werden die zerkleinerten Früchte bevorzugt. Es können aber auch Stiele verarbeitet werden da die Wirkstoffe auch in diesen Pflanzenteilen enthalten sind.

Reiner Naturjoghurt aus Milch von Kühen, Schafen, Ziegen anderen Tieren oder aus Gemischen von verschiedenen Milcharten, wird ebenfalls mit Wasser bei 10 bis 30 °C, vorzugsweise 15-25 °C, extrahiert über einen Zeitraum von 5 bis 24 Stunden. Anschließend wird die wäßrige Phase z.B. durch Filtration abgetrennt und ein Extraktionsrückstand gewonnen. Dieser Extraktionsrückstand wird dann einer Sterilisation unterworfen. Die im Rückstand enthaltenen Proteine, Milchfette, Zucker, Folsäure, Biotin, teilweise Vitamine bleiben für die Weiterverwendung erhalten. Sie werden auch nicht durch die Enzyme des Ananas-Extraktes abgebaut, da diese in verkapselter Form vorliegen.

In ein wäßriges Gel werden der verkapselte Ananas-Extrakt und der Joghurtextraktrückstand gleichzeitig eingemischt. Dabei entsteht ein stabiler Basiskomplex als Wirkstoff-Ausgangsmaterial für kosmetische Formulierungen.

Zu geeigneten Gelbildnern gehören Carbomer, Xanthangummi, Carrageenan, Akaziengummi, Guargummi, Agar-Agar, Alginate und Tylosen, Carboxymethylcellulose, Hydroxyethylcellulose, bestimmte Polyacrylate, Polyvinylalkohol, Polyvinylpyrrolidon, Montmorillonit. Bevorzugt sind carbomer, Guargummi und Agar.

Der erfindungsgemäße Komplex kann mit üblichen kosmetischen Hilfsstoffen und Trägerstoffen kombiniert werden, wie sie generell in solchen Zubereitungen verwendet werden, z.B. Wasser, Konservierungsmittel, Vitamine, Farbstoffe, Pigmente mit färbender Wirkung, Radikalfänger, Verdickungsmittel, weichmachende Substanzen, feuchthaltende Substanzen, Duftstoffe, Alkohole, Polyole, Ester, Elektrolyte, Gelbildner, polare und unpolare Öle, Polymere, Copolymere, Emulgatoren, Wachse, Stabilisatoren, Glycerin. Dabei können auch übliche O/W-Emulsionen oder W/O-Emulsionen mit diesem Basiskomplex hergestellt werde, wobei als Öle alle üblichen Öle wie Mineralöle, synthetische Öle, Siliconöle sowie pflanzliche Öle, wie Calendulaöl, Jojobaöl, Avocadoöl, Macadamianußöl, Rizinusöl, Weizenkeimöl, Traubenkernöl, Kukuinußöl, Distelöl, Nachtkerzenöl, Safloröl oder ein Gemisch mehrerer davon verwendet werden können.

Außerdem können weitere Wirksubstanzen zugesetzt werden, wie Kaolin gemäß WO96/17588, der mit sphärischen TiO₂- oder SiO₂-Teilchen mit einer Teilchengröße <5 µm modifiziert ist, wobei die sphärischen Teilchen einen Anteil an der Kaolinmischung von 0,5 bis 10 Gew-% haben. Man erhält damit ein sehr weiches Hautgefühl und eine zusätzliche entzündungswidrige Wirksamkeit.

Weitere Wirkstoffe sind asymmetrische lamellare Aggregate, wobei diese Aggregate aus Phospholipiden und mit Sauerstoff beladenem Fluorcarbon oder Fluorcarbongemisch bestehen und einen Gehalt an Fluorcarbon im Bereich von 0,2 bis 100 % Gewicht/Volumen haben, wobei das Phospholipid einen Phosphatidylcholingehalt von mehr als 30 bis 99 Gewichts-% hat gemäß WO94/00098.

Zu weiteren kosmetischen Wirkstoffen gehören z. B. Aufschlußprodukte von Hefen oder pflanzlichen Stoffen, hergestellt durch ein schonendes Ultraschall-Aufschlußverfahren gemäß WO 94/13783; ein im wäßrigen Medium mild abgebautes und mit einer unterstöchiometrischen Menge eines C₁₀-C₂₀-Fettsäurehalogenids kondensiertes Produkt aus pflanzlichem oder tierischem Ausgangsmaterial gemäß WO96/29048; eine Wirkstoffkombination aus a) einem Produkt eines enzymatischen Extraktionsprozesses des maritimen Planktons Artemia salina, wobei das Extraktionsprodukt aus phosphorylierten Nucleotiden mit der Hauptkomponente Diguanosin-tetraphosphat besteht; b) D-myo-Inosit-1,4,5-triphosphat; c) Glycan; wobei das Verhältnis a:b:c im Bereich 1 : 0,1-50 : 0,1-30 liegt,gemäß WO99/38483; oder einer kosmetischen Wirkstoffzubereitung mit hohem Radikalschutzfaktor gemäß WO99/66881.

Das Kosmetikum kann auch einen Gehalt an Schellack in der wäßrigen Phase aufweisen, wobei der Anteil an reinem Schellack in einer O/W-Emulsion im Bereich von 0,1 bis 20 Gew-%, in einer W/O-Emulsion im Bereich von 0,1 bis 15 Gew-%, in einem Hydrogel im Bereich von 0,1 bis 10 Gew-% liegt. Die Herstellung dieses wasserlöslichen Schellacks erfolgt gemäß WO99/06010.

Die Verwendung des erfindungsgemäßen kosmetischen Basiskomplexes kann z.B. erfolgen in Sonnencremes, Sonnengelen, Aftersun-Produkten, Tagescremes, Nachtcremes, Masken, Körperlotionen, Reinigungsmilch, Make up's, Augenkosmetik, Haarmasken, Haarspülungen, Haarshampoos, Duschgelen, Duschölen, Badeölen, Lotionen, Gelen. Die Herstellung derartiger Produkte erfolgt auf eine Weise, wie sie dem Fachmann auf diesem Gebiet bekannt ist.
Der Anteil der beiden Komplexbildner liegt vorteilhaft im Bereich von 40:60 bis 60:40.

Der Komplex kann in einer kosmetischen Formulierung in einem Anteil von 0,5 bis 10 Gew-%, bezogen auf die gesamte Formulierung, vorliegen.

Der erfindungsgemäße Basiskomplex enthält wertvolle Wirkstoffe, die nach dem Auftragen des Kosmetikums auf die Haut ihre Wirkung über einen längeren Zeitraum entfalten können. Wesentliche spezielle Wirkung ist - in Abhängigkeit von der aufgetragenen Menge bzw. von der Größe der für die Behandlung vorgesehenen Körperfläche - ein "Happyness"-Gefühl. Darunter ist nicht ein reines Glücksgefühl zu verstehen, sondern eine ausgewogene Zufriedenheit, eine Entspannung gepaart mit angenehmen Empfindungen. Ohne an eine Theorie gebunden zu sein, scheint sich die Aufnahme des Produktes über die Haut auf den Serotoninspiegel und die Noradrenalinbildung auszuwirken, nicht zuletzt durch die Zuführung von Folsäure.

Weiterhin wird durch den Gehalt des Enzyms Bromelin, der durch Zusätze in einen Bereich von 0,1-1 Gew-%, bezogen auf die gesamte kosmetische Formulierung, gebracht werden kann, eine sanfte Peeling-Wirkung erzielt, d.h. unauffällige, gleichmäßige und sanfte Abtragung oberer Hautpartikel.

Ein weiterer Wirkaspekt ist die durch die Ananas-Extraktion bedingte helle Gelbfärbung des Extraktes und des Basiskomplexes. Es ist bekannt, daß auch mit einer solchen hellen Gelbfärbung eine Stimulierung von Glücksgefühlen einhergeht, so daß eine bevorzugte Färbung einer kosmetischen Formulierung, enthaltend den Basiskomplex, die Farbe Hellgelb ist.

Die Erfindung soll nachstehend durch Beispiele näher erläutert werden. Alle Angaben erfolgen in Gewichtsprozent, sofern nichts anderes angegeben ist.

### Beispiel 1

| Emulsion | |
|---|---|
| Phase A | |
| Polyglyceryl-4 AlmondateSHEA Butterate | 3,5 |
| Paradecinol FV4.7 | 18 |

| Phase B | |
|---|---|
| Wasser | ad 100 |
| Glycerin | 3 |
| Magnesiumsulfat | 0,5 |

| Phase C | |
|---|---|
| Basiskomplex mit Ananasextrakt:Joghurtextrakt- | |
| rückstand = 80:20 | 0,5 |
| Parfüm | 0,5 |
| Konservierungsmittel | 0,8 |

Die Herstellung von Phase A und B erfolgt jeweils getrennt bei ca. 80 ° durch Vermischen der Einzelbestandteile. Dann werden beide Phasen miteinander vermischt. Nach dem Abkühlen auf etwa 40 °C wird die Phase C unter Rühren hinzugegeben.

### Beispiel 2

| Gel | |
|---|---|
| Phase A | |
| Wasser | ad 100 |
| Carbomer | 1,5 |

| Phase B | |
|---|---|
| Mineralöl | 2 |
| Babassuöl | 3 |
| EDTA | 0,1 |

| Phase C | |
|---|---|
| Triethanolamin | 1,5 |

| Phase D | |
|---|---|
| Basiskomplex mit Ananasextrakt:Joghurtextrakt- | |
| rückstand = 20:80 | 7 |
| Parfüm | 0,5 |
| Konservierungsmittel | 1 |

Die Phase A wird durch Mischen der Einzelbestandteile und sorgfältigem Rühren bei etwa 60 °C hergestellt, danach die Phase B in gleicher Weise bei etwa 65 °C. Durch Zugabe der Phase C erfolgt eine Neutralisierung, und bei etwa 40 °C wird die Phase D unter Rühren zugegeben.

### Beispiel 3

Die Herstellung des Basiskomplexes erfolgt durch Vermischen etwa gleicher Gewichtsteile Wasser und frischer, geschälter und zerkleinerter Ananasstücken. Das Mischen wird über einen Zeitraum von 18 Stunden bei 20 °C fortgeführt. Anschließend wird das Gemisch filtriert und das Filtrat mit Phospholipiden bei Raumtemperatur vermischt, um eine Verkapselung zu erreichen.

Weiterhin werden 30 Masseteile Wasser und 70 Masseteile eines frischen Naturjoghurt aus Kuhmilch bei 20 °C für einen Zeitraum von 20 Stunden unter intensivem Rühren miteinander vermischt und das Gemisch anschließend abfiltriert. Das Filtrat wird verworfen und der Rückstand für die Herstellung des Basiskomplexes eingesetzt.

In ein wäßriges Gel (Carbomer) werden gleiche Anteile als Liposomen vorliegender Ananas-Extrakt und Joghurtextraktrückstand bei ca. 20 °C zusammen eingerührt und der dabei erhaltene Basiskomplex als Ausgangsmaterial für eine kosmetische Formulierung verwendet. Der Basiskomplex enthält ca. 80 Gew-% Extrakte und ca 20 Gew-% wäßriges Gel.

## Patentansprüche

1. Kosmetischer Basiskomplex, **dadurch gekennzeichnet, daß** er enthält
- eine wäßrige Gelgrundlage;
- in der wäßrigen Gelgrundlage ein verkapselter Extrakt einer wäßrigen Extraktion bei 10-30 °C der Ananas-Frucht;
- in der wäßrigen Gelgrundlage der Rückstand, der bei einer wäßrigen Extraktion bei 10-30 °C von Joghurt anfällt;
und wobei das Verhältnis Ananas-Extrakt zu Joghurtextraktrückstand im Bereich von 20:80 bis 80:20 liegt.

2. Basiskomplex nach Anspruch 1, **dadurch gekennzeichnet, daß** der Ananas-Extrakt in Liposomen verkapselt ist.

3. Basiskomplex nach Anspruch 2, **dadurch gekennzeichnet, daß** die Liposomen Phospholipid-Liposomen sind.

4. Basiskomplex nach Anspruch 1, **dadurch gekennzeichnet, daß** das Verhältnis Ananas-Extrakt zu Joghurtextraktrückstand im Bereich von 40:60 bis 60:40 liegt.

5. Basiskomplex nach Anspruch 1, **dadurch gekennzeichnet, daß** der Komplex einen Anteil von Bromelin im Bereich von 0,1 bis 1 Gew-% aufweist.

6. Basiskomplex nach Anspruch 1, **dadurch gekennzeichnet, daß** der Komplex in einer kosmetischen Formulierung vorliegt in einem Anteil von 0,5 bis 10 Gew-%, bezogen auf die gesamte Formulierung.

7. Basiskomplex nach Anspruch 6, **dadurch gekennzeichnet, daß** die kosmetische Formulierung weitere Bestandteile enthält, ausgewählt aus der Gruppe, bestehend aus Ölen, Estern, Feuchthaltemitteln, Lösungsmitteln, Pigmenten, Emulgatoren, UV-Filtern, Antioxidationsmittel, Vitaminen, Gelbildnern, weiteren Wirkstoffen und Gemischen davon.

8. Basiskomplex nach Anspruch 6, **dadurch gekennzeichnet, daß** die kosmetische Formulierung in Form von Cremes, Gelen, Duschbädern, Sonnenprodukten, Lotionen, O/W-Emulsionen, W/O-Emulsionen oder Make-up's vorliegt.

9. Basiskomplex nach Anspruch 6, **dadurch gekennzeichnet, daß** die kosmetische Formulierung eine helle Gelbfärbung aufweist.

10. Verfahren zur Herstellung eines kosmetischen Basiskomplexes, **dadurch gekennzeichnet, daß** ein bei 10 bis 30 °C erhaltener wäßriger Extrakt von Ananas nach einer Filtration in Liposomen verkapselt wird und zusammen mit dem Rückstand einer bei 10 bis 30 °C durchgeführten wäßrigen Extraktion von Joghurt in ein kosmetisches Gel eingemischt wird.

## Claims

1. A cosmetic base complex which comprises
- an aqueous gel base;
- in the aqueous gel base, an encapsulated extract from an aqueous extraction of pineapple fruit at a temperature of 10-30 °C;
- in the aqueous gel base, the residue obtained by an aqueous extraction of yogurt at a temperature of 10-30 °C;
the ratio of pineapple extract to yogurt residue ranging from 20:80 to 80:20.

2. A base complex according to Claim 1 wherein the pineapple extract is encapsulated in liposomes.

3. A base complex according to Claim 2 wherein the liposomes are liposomes formed by phosholipids.

4. A base complex according to Claim 1 wherein the ratio of pineapple extract to yogurt residue is in the range from 40:60 to 60:40.

5. A base complex according to Claim 1 wherein the complex contains bromelin in the range from 0.1 to 1 % by weight.

6. A base complex according to Claim 1 wherein the said complex is contained in a cosmetic formulation in an amount ranging from 0.5 to 10 % by weight relative to the total weight of the formulation.

7. A base complex according to Claim 6 wherein the cosmetic formulation contains further components selected from the group consisting of oils, esters, moisturizing substances, solvents, pigments, emulsifiers, UV filters, antioxidants, vitamins, gel forming agents, further active agents and mixtures thereof.

8. A base complex according to Claim 6 wherein the cosmetic formulation is present in the form of creams, gels, shower products, sun products, lotions, O/W emulsions, W/O emulsions or make ups.

9. A base complex according to Claim 6 wherein the cosmetic formulation has a bright yellow colour.

10. Method for producing a cosmetic base complex which comprises the steps of producing an aqueous extract from pineapple at a temperature ranging from 10 to 30 °C, subsequently filtering the said extract and encapsulating it in liposomes and mixing it into a cosmetic gel together with the residue of an aqueous extraction of yogurt carried out at a temperature ranging from 10 to 30 °C.

## Revendications

1. Complexe de base cosmétique, **caractérisé en ce qu'**il contient :
- une base aqueuse de gel ;
- dans la base aqueuse de gel, un extrait encapsulé d'une extraction aqueuse à 10-30°C du fruit d'ananas ;
- dans la base aqueuse de gel, le résidu qui se forme lors d'une extraction aqueuse à 10-30°C du yogourt, et
où le rapport de l'extrait d'ananas au résidu d'extrait de yogourt se situe dans l'intervalle allant de 20:80 à 80:20.

2. Complexe de base selon la revendication 1, **caractérisé en ce que** l'extrait d'ananas est encapsulé dans des liposomes.

3. Complexe de base selon la revendication 2, **caractérisé en ce que** les liposomes sont des liposomes de phospholipide.

4. Complexe de base selon la revendication 1, **caractérisé en ce que** le rapport de l'extrait d'ananas au résidu d'extrait de yogourt se situe dans l'intervalle allant de 40:60 à 60:40.

5. Complexe de base selon la revendication 1, **caractérisé en ce que** le complexe présente une quantité de broméline dans l'intervalle allant de 0,1 à 1% en poids.

6. Complexe de base selon la revendication 1, **caractérisé en ce que** le complexe est présent dans une formulation cosmétique en une quantité de 0,5 à 10% en poids, sur base de la formulation totale.

7. Complexe de base selon la revendication 6, **caractérisé en ce que** la formulation cosmétique contient d'autres constituants, choisis parmi le groupe consistant en les huiles, les esters, les agents humidifiants, les solvants, les pigments, les émulsionnants, les filtres U.V., les antioxydants, les vitamines, les agents formant gel, d'autres agents actifs et leurs mélanges.

8. Complexe de base selon la revendication 6, **caractérisé en ce que** la formulation cosmétique se présente comme une crème, un gel, un bain-douche, un produit solaire, une lotion, une émulsion H/E, une émulsion E/H ou un maquillage.

9. Complexe de base selon la revendication 6, **caractérisé en ce que** la formulation cosmétique présente une couleur jaune clair.

10. Procédé de préparation d'un complexe de base cosmétique, **caractérisé en ce qu'**un extrait aqueux obtenu de 10 à 30°C, d'ananas est encapsulé après filtration, dans des liposomes et est incorporé avec le résidu d'une extraction aqueuse réalisée de 10 à 30°C de yogourt dans un gel cosmétique.
